# FASCICULE DE BREVET EUROPEEN

(11) **EP 2 121 724 B1**
(45) Date de publication et mention de la délivrance du brevet: **03.08.2011**
(21) Numéro de dépôt: 07871830.1
(22) Date de dépôt: 11.12.2007
(51) Int. Cl.: C07J 41/00

(54) **NOUVEAU PROCEDE POUR L'OBTENTION DIASTEREOSELECTIVE D'UNE AMINE PRIMAIRE CHIRALE SUR UN STEROÏDE**
NEUES VERFAHREN ZUR DIASTEREOSELEKTIVEN HERSTELLUNG EINES CHIRALEN PRIMÄRAMINS AUF EINEM STEROID
NOVEL METHOD FOR THE DIASTEREOSELECTIVE PRODUCTION OF A CHIRAL PRIMARY AMINE ON A STEROID

(30) Priorité: 13.12.2006 FR 0610853
(43) Date de publication de la demande: 25.11.2009
(73) Titulaire: SANOFI, 75013 Paris (FR)
(72) Inventeur: BERNARD, Daniel, F-75013 Paris (FR); BOUSQUET-FRANCES, Joëlle, F-75013 Paris (FR); CAZENAVE, Gérard, F-75013 Paris (FR); ODDON, Gilles, F-75013 Paris (FR); SIMONNET, André, 75013 PARIS (FR)
(74) Mandataire: Weber, Mathieu
(86) Numéro de dépôt international: PCT/FR2007/002035
(87) Numéro de publication internationale: WO 2008/090272

(56) Documents cités:
- US-A- 3 035 066
- US-A- 4 197 296
- MATYAS, LIBOR ET AL: "Series on steroids. Part CDXVI: Effects of 3.alpha.-amino-5.alpha.-pregnan- 20-one on GABAA receptor: Synthesis, activity and cytotoxicity" COLLECTION OF CZECHOSLOVAK CHEMICAL COMMUNICATIONS , 69(7), 1506-1516 CODEN: CCCCAK; ISSN: 0010-0765, 2004, XP002447589
- DAVIS P.A ET AL.: "The "Triamino-analogue" of Methyl Cholate; A Practical, Large-Scale Synthesis" SYNLETT, vol. S1, 1999, pages 991-993, XP002447590

## Description

La présente invention a pour objet un procédé pour l'obtention diastéréosélective d'une amine primaire sur un stéroïde.

Le procédé selon l'invention est particulièrement intéressant car il permet le développement de nouvelles voies de synthèses de stéroïdes incluant l'obtention diastéréosélective d'amines chirales, à échelle industrielle.

Des voies de synthèses permettant l'introduction d'une amine primaire sur un stéroïde sont décrites. Elles impliquent généralement une réduction d'oximes substituées ou non par l'action d'agents réducteurs tels que des hydrures, le zinc dans l'acide acétique, le sodium dans un alcool. Ces procédés conduisent le plus souvent à des mélanges dans des proportions diverses d'isomères alpha et béta de l'amine. Les diastéréoisomères doivent très souvent être isolés par chromatographie préparative. Ainsi ces voies de synthèse ne permettent pas la transposition au niveau industriel.

Parmi les documents de l'art antérieur, on peut citer plus particulièrement la demande WO 01/83512 qui décrit la préparation de stéroïdes portant un groupe amino, obtenu dans des conditions stéréosélectives, par réduction d'un groupe azido de configuration appropriée, à l'aide d'un hydrure ou d'hydrogène en présence d'un catalyseur au palladium. Le document US 3035066 décrit la réduction d'une fonction oxime substituée en position 3 et 11 d' un composé dérivé de la pregnanone en une fonction amine primaire en présence de sodium dans l'éthanol.

La transposition à l'échelle industrielle de telles techniques, notamment celle mettant en oeuvre un intermédiaire azido est très difficile, voire impossible suivant les sites industriels. On sait en effet que l'utilisation de la chimie des azotures à l'échelle industrielle nécessite la construction d'installations spécifiques. Les azotures produisent de l'acide hydrazoïque (ou azoture d'hydrogène) en présence de traces d'acides, lequel. est un gaz très toxique et très explosif. Les azotures de métaux lourds sont également très explosifs, il faut donc éviter tout contact des azotures avec des alliages contenant des métaux lourds. Ceci implique donc des mesures de sécurité adaptées et lourdes pour la mise en oeuvre des réactions à l'azoture de sodium.

La publication J. Chem. Research (5) 2003, 234-235 rappelle que des 3-aminostéroïdes ont été obtenus à partir de dérivés 3-hydroxy par tosylation, formation d'azides et réduction en amines. Elle ajoute que cette méthode conduit à une inversion de la configuration en 3. Elle décrit une méthode alternative en 4 stades ne provoquant par d'inversion de configuration, consistant essentiellement à traiter une cétone en 3 par l'hydroxylamine dans la pyridine, à réduire l'oxime par le sodium dans l'isopropanol et à traiter le milieu réactionnel par l'acide acétique.

La publication Bull. Soc. Chim. 1971, n°11 p. 4072-4078 rappelle les différentes méthodes d'accès aux 3-amino stéroïdes connues à l'époque consistant à réduire des composés 3-oximino (Na-alcool, LiAlH₄-éther/dioxanne, complexe LiAlH₄-AlCl₃) et précise que ces méthodes ne donnent accès que difficilement aux amines primaires par suite des difficultés pour séparer le composé dérivé des mélanges complexes obtenus. Des mélanges d'isomères en 3 ainsi que des composés de dégradation/transposition sont obtenus. Deux autres méthodes plus stéréospécifiques de réduction de composés 3-oximino sont décrites, l'hydrogénation catalytique en présence de platine d'Adams et l'action du lithium dans l'éthylamine. Cette dernière conduit à un mélange d'amines majoritairement β, dont il est donc nécessaire de séparer l'isomère α, par cristallisation ou chromatographie.

La demanderesse a mis au point un nouveau procédé totalement stéréosélectif de préparation d'amines primaires stéroïdiennes au départ d'oximes, permettant une transposition aisée à l'échelle industrielle et d'application générale, dès lors que la molécule ne comporte pas par ailleurs de substitution sensible aux conditions de la réaction.

La présente invention a ainsi pour objet un procédé de préparation stéréosélectif d'amines primaires stéroïdes de configuration α ou β, en position 1, 2, 3, 4, 6, 7, 11, 12, 15, 16 ou 17 sur le squelette stéroïde, caractérisé en ce que l'on traite un oxime de formule (II) : 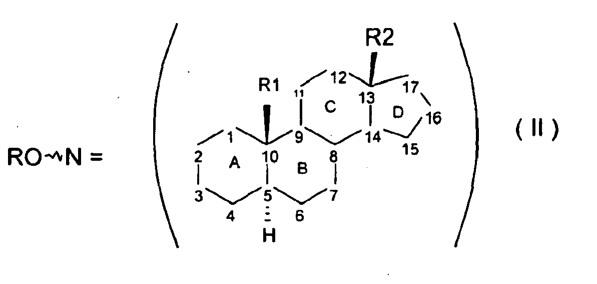 dans laquelle R représente un atome d'hydrogène, un radical alkyle linéaire, ramifié ou cyclique renfermant de 1 à 12 atomes de carbone, ou un radical aryle ou aralkyle renfermant jusqu'à 12 atomes de carbone, R1 représente un atome d'hydrogène ou un radical alkyle inférieur contenant de 1 à 4 atomes de carbone, R2 représente un radical alkyle inférieur contenant de 1 à 4 atomes de carbone, la fonction oxime est située en position 1, 2, 3, 4, 6, 7, 11, 12, 15, 16 ou 17 sur le squelette, lequel peut être substitué par ailleurs par un ou plusieurs groupes non sensibles aux conditions de la réaction définies ci-après,
avec du lithium métal dans l'ammoniac liquide, à une température comprise entre -33°C et -90°C, dans un mélange d'un solvant de type éther et d'un alcool aliphatique, et obtient le composé attendu de formule (I) : 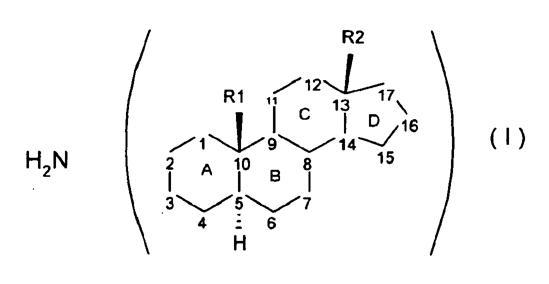 dans laquelle l'amine de configuration α ou β est dans la position correspondant à celle de l'oxime sur le composé de formule (II).

L'amine obtenue est en configuration équatoriale, ce qui correspond à la position thermodynamiquement la plus stable.

Les groupes sensibles aux conditions de la réaction dont il est question plus haut sont bien connus du chimiste organicien, mais comme indiqué, le procédé selon l'invention est d'application générale et en raison de la réactivité de l'oxime dans les conditions de réduction utilisées, la mise en oeuvre d'une quantité déterminée de lithium avec un suivi de la réaction et une interruption de celle-ci lorsque l'oxime a disparu, permet d'éviter de toucher d'autres groupes réputés sensibles tels les groupes esters, amides, cétone, voire les substituants aromatiques, ou les doubles liaisons.

Les groupes qui ne peuvent être présents sont essentiellement les énones conjuguées.

La quantité de lithium utilisée est au moins la quantité théorique minimale de 4 équivalents mais, ainsi que le sait l'homme du métier, une quantité supérieure peut être nécessaire, en particulier si la molécule renferme un ou plusieurs protons labiles, entraînant une consommation de lithium.

L'invention a notamment pour objet un procédé tel que défini précédemment, caractérisé en ce que l'on opère à une température comprise entre -50°C et -80°C.

L'invention a notamment pour objet un procédé tel que défini précédemment caractérisé en ce que l'alcool utilisé est un alcanol renfermant de 1 à 6 atomes de carbone, linéaire, ramifié ou cyclique, éventuellement substitué par un ou plusieurs atomes de fluor.

L'invention a plus particulièrement pour objet un procédé tel que défini précédemment, caractérisé en ce que l'alcool utilisé est un alcanol renfermant de 1 à 4 atomes de carbone, linéaire ou ramifié, éventuellement substitué par un ou plusieurs atomes de fluor.

L'invention a notamment pour objet un procédé tel que défini précédemment, caractérisé en ce que le solvant de type éther utilisé est le tétrahydrofuranne ou le méthyltétrahydrofuranne. Toutefois, les autres éthers connus de l'homme du métier, liquides dans les conditions de la réaction, sont utilisables selon l'invention.

R peut être tout radical alkyle, aryle ou aralkyle tel que défini plus haut, mais l'invention a notamment pour objet un procédé caractérisé en ce que R représente un radical méthyle, éthyle ou benzyle.

Ainsi qu'il est indiqué plus haut, le procédé de l'invention est d'application générale et celle-ci a notamment pour objet un procédé tel que défini précédemment, caractérisé en ce que le squelette stéroïde mis en jeu est substitué par un ou plusieurs éléments choisis dans le groupe constitué par halogène, cétone libre ou protégée, hydroxy libre ou sous forme éthérifié, amino, carboxy, carboxy estérifié, imide, amide, une chaîne carbonée mono ou divalente saturée ou insaturée, linéaire, ramifiée ou cyclique, renfermant jusqu'à 15 atomes de carbone, le cas échéant interrompue par 1 à 3 atomes d'oxygène, de soufre ou d'azote, et éventuellement substituée par hydroxy ou cétone libre ou protégée, halogène, carboxy, carboxy estérifié et comporte, le cas échéant, une ou plusieurs doubles liaisons dans les cycles A et/ou B et/ou C et/ou D, conjuguées ou non.

Lorsque le squelette stéroïde est substitué par plusieurs éléments choisis dans le groupe défini plus haut, il peut s'agir de plusieurs fois le même élément, par exemple halogène, cétone ou hydroxy, libre ou protégé.

Par halogène, on entend de préférence le fluor.

Par groupe hydroxy éthérifié, on entend toutes protections usuelles connues du chimiste, qu'il s'agisse de la protection d'un groupe hydroxy ou de celle de deux groupes hydroxy fixés sur deux carbones adjacents du squelette.

On peut citer par exemple les éthers clivables tels que ceux formés avec un groupe C(₁₋₆) alcoyle, notamment méthyle ou t-butyle, avec un groupe C (₁₋₆) alcoyl-phényle, notamment benzyle, p-méthoxybenzyle, p-nitrobenzyle, les éthers d'allyle, trityle, méthoxyméthyle, méthoxyethoxyméthyle, éthoxyéthyle, tétrahydropyranyle, les éthers silylés, notamment les éthers de triméthyle, triéthyle ou triisopropylsilyle, de t-butyldiméthylsilyle ou de diméthylarylsilyle.

On peut encore citer les esters clivables tels que ceux formés avec un groupe acétyle, benzoyle, phénylacétyle, formyle, haloacétyle, tel que choracétyle, di ou trichloracétyle, ou trifluoroacétyle.

On peut encore citer les carbonates, ainsi que les cétals cycliques tels que
-O-(CH₂)m-O-, -O-(CH₂)m-S-, -S-(CH₂)m-S-, -O-CH₂-C(C₁-₄ alcoyl)₂-CH₂-O-, ou encore les cétals acycliques tels que -(CH₃O)₂- ou-(EtO)₂-, m étant de préférence 1, 2 ou 3

Par protection du groupe cétone, on entend toute protection connue du chimiste et notamment les cétals et thiocétals cités ci-dessus.

Par amino, on entend amino primaire, secondaire ou tertiaire, notamment C(₁₋₆) alcoyl ou dialcoylamino.

Par carboxy estérifié, on entend notamment un ester de C(₁₋₆)alcoyle.

La chaîne carbonée peut être toute chaîne, connue dans le domaine stéroïde, notamment, alcoyle linéaire, ramifié ou cyclique, alcényle, alcynyle ou alcoylène, interrompu pour 1 à 3 hétéroatomes et/ou substitué comme indiqué précédemment.

Comme exemple de chaîne alcoyle linéaire ou ramifiée de 1 à 12 atomes de carbone on peut citer notamment méthyle, éthyle, propyle, butyle, pentyle, hexyle, octyle et leurs isomères ramifiés tels que isopropyle, isobutyle, isopentyle, neo-pentyle, isohexyle, 3-méthylpentyle, sec-butyle, tert-butyle, tert-pentyle.

Comme exemple de chaîne alcoyle cyclique, on peut citer notamment cyclopropyle, cyclobutyle, cyclopentyle, cyclohexyle, cycloheptyle ou cyclooctyle, éventuellement substitué, par exemple par un groupement alcoyle renfermant 1 à 4 atomes de carbone.

Comme exemple de chaîne alcényle, on peut citer notamment vinyle, allyle ou butényle.

Comme exemple de chaîne alcynyle, on peut citer notamment éthynyle ou propargyle.

Comme exemple de chaîne alcoylène, on peut citer notamment méthylène ou toute chaîne divalente dérivée des alcoyles ci-dessus. Cette chaîne peut, le cas échéant, être fixée sur deux carbones adjacents du squelette et également former un système bicyclique.

Lorsque les cycles renferment une ou plusieurs doubles liaisons, celles-ci sont notamment en position 1(2), 3(4), 1,3, 5, 5(6), 6(7), 9(11), 15(16) ou 16(17).

L'invention a notamment pour objet un procédé tel que défini précédemment, caractérisé en ce que lorsque le stéroïde est substitué par une chaîne alcoylène, celle-ci n'est pas un méthylène en 17.

L'invention a plus particulièrement pour objet un procédé tel que défini précédemment, caractérisé en ce que le squelette du stéroïde mis en jeu est substitué par un ou plusieurs éléments choisis dans le groupe constitué par fluor, cétone libre ou protégée, hydroxy libre ou protégé, amino, éther, amide, imide, les chaînes alcoyle, alcényle, alcynyle et alcoylène telles que définies plus haut, et comporte, le cas échéant, une ou plusieurs doubles liaisons dans les cycles A et/ou B et/ou C et/ou D, conjuguées ou non.

L'invention a tout particulièrement pour objet un procédé tel que défini précédemment, caractérisé en ce que le squelette du stéroïde mis en jeu est substitué par un ou plusieurs éléments choisis dans le groupe constitué par cétone libre ou protégée, hydroxy libre ou protégé et une chaîne alcoyle linéaire ou ramifiée, renfermant jusqu'à 12 atomes de carbone, et comporte, le cas échéant, une ou deux doubles liaisons dans les cycles A et/ou B et/ou C et/ou D.

La présente invention est illustrée par les exemples qui suivent :

### Exemple 1 : Synthèse de la 3-β amino stanolone

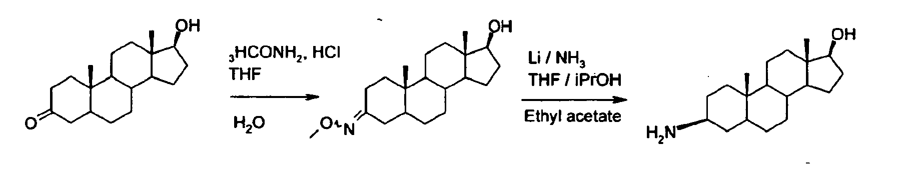

### Préparation de 3-Z,E-méthyl oxime de stanolone

30g de Stanolone sont dissous dans 120 ml de tétrahydrofuranne, à température ambiante. On ajoute 21.54ml de triéthylamine (1.5éq) et 18.8 ml de soude aqueuse à 32% m/m (2éq) à cette solution, à 20°C. On ajoute sous vive agitation 47.6 ml de chlorhydrate de méthylhydroxylamine en solution aqueuse à 31 % m/m (1.9éq). On agite 3h à 20°C puis 2h à 60°C la solution biphasique et vérifie la fin de réaction par CCM. On distille sous vide à 60°C, en gardant un volume réactionnel constant par ajout d'eau, jusqu'à ce que l'indice de réfraction du distillat soit celui de l'eau.

Le produit cristallise pendant l'échange de solvants. On agite 16h à 20°C puis on ajoute 180 ml d'eau à la suspension, à 20°C. On agite 30 minutes à cette température, puis on essore le solide et le lave à l'eau. On sèche le produit à 40°C pendant 18h.

On obtient 33.3g de 3-Z,E-méthyl oxime de stanolone (rdt = 100%).

Spectre de R.M.N.: 1H à 300 MHz, DMSO d6 référencé à 2,52 ppm. δ en ppm. Mélange 50/50 des isomères Z et E

| δ ¹H obs. (ppm) | Multiplicité J (en Hz) |
|---|---|
| 0.59-0.70 | m, 1H |
| 0.65 | s, 3H |
| 0.75-1.43 | m, 11.5H |
| 0.86 | s, 3H |
| 1.43-2.08 | m, 8H |
| 2.16 | m, 0.5H |
| 2.76 | dd, 0.5H (J=15.4 et 3.4) |
| 2.98 | m, 0.5H |
| 3.44 | m, 1H |
| 3.69 | S large, 3H |
| 4.41 | d, (1H J=4.8) |

Spectre de masse : Electro Spray Ionisation en mode Positif.

MH+= (m/z) = 320

### 3-β-aminostanolone

20g de 3-Z,E-méthyl oxime de stanolone sont mis en suspension dans 200ml de tétrahydrofuranne et 25ml d'isopropanol. On ajoute cette suspension sur 100ml d'ammoniac liquide à -50°C. On refroidit à -70°C et ajoute 1.942g de lithium (4.47éq) en granules, en 9 fractions. On maintient sous agitation pendant 1 heure, puis on traite la réaction par ajout de 16.7g de chlorure d'ammonium (5éq). On laisse ensuite la suspension se réchauffer à température ambiante, ajoute à 20°C 40ml d'eau puis distille sous vide à 60°C, en gardant un volume réactionnel constant par ajout régulier d'eau, jusqu'à ce que l'indice de réfraction du distillat soit proche de celui de l'eau. La suspension aqueuse est refroidie à 20°C et extraite par 200ml de chlorure de méthylène: On filtre un insoluble et on évapore à sec la phase organique. On obtient 6.95g d'extrait sec.

D'autre part, l'insoluble est repris dans 700ml de chlorure de méthylène et 550ml d'eau et le pH est ajusté à 12.5 par l'introduction de 2 ml de soude aqueuse à 32% m/m. La phase organique est évaporée à sec et on obtient 10.4g de produit supplémentaire. Les 2 produits sont regroupés, soit 17.35g, rendement = 95.1%

5g de ce produit sont mis en suspension dans 150ml d'acétate d'éthyle pendant 1h à 20°C, le produit est filtré et lavé à l'acétate d'éthyle.

On obtient 2.5g de 3-β-amino stanolone pure.

Spectre de R.M.N. : 1H à 400 MHz, CDC13 d1 référencé à 7,27 ppm.

| δ ¹H obs. (ppm) | Multiplicité J (en Hz) | δ ¹H obs. (ppm) | Multiplicité J (en Hz) |
|---|---|---|---|
| 0.64 | m, 1H | 2.05 | m, 1H |
| 0.73 | s, 3H | 2.64 | m, 1H |
| 0.80 | s, 3H | 3.62 | t, 1H (J=8.0) |
| 0.87 | m, 1H | | |
| 0.92-1.15 | m, 5H | | |
| 1.17-1.32 | m, 5H | | |
| 1.32-1.53 | m, 3H | | |
| 1.53-1.62 | m, 2H | | |
| 1.63-1.73 | m, 3H | | |
| 1.79 | dt, 1H (J=12.6 et 3.2) | | |

Rapport α/β de l'amine primaire = 0/100

Spectre de masse : Electro Spray Ionisation en mode Positif. MH+ (m/z) = 292.

### Exemple 2 : 17-β -amino DHEA

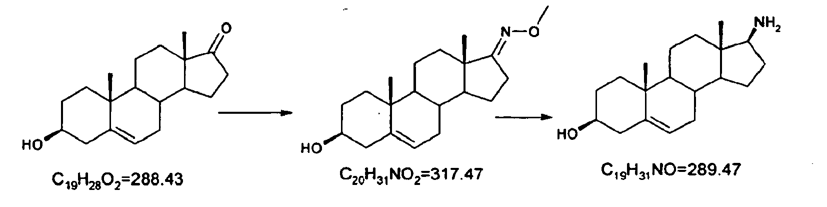

### Préparation de 17-E-méthyl oxime de DHEA

50g de DHEA sont mis en solution dans 200 ml de toluène et 124 ml de pyridine (8.9éq). On ajoute à 20°C, 16.25g de chlorhydrate de méthoxylamine (pureté 98%, soit 1.1 éq.). On agite 5h à 50°C puis 48h à 30°C. On essore et lave à l'eau, puis sèche le solide sous vide.

D'autre part, on décante le filtrat et lave la phase organique avec 2 fois 500 ml d'eau. On distille la phase organique à sec sous vide. L'extrait sec et les cristaux précédemment filtrés sont mélangés ensemble. On obtient 53.07g de 17-E-méthyloxime de DHEA, soit un rendement total de 96.4%.

Spectre de R.M.N. : 400 MHz, CDCl₃ d1.

| Numéro | δ ¹H obs. | Multiplicité J (en Hz) | δ ¹³C obs. | Numéro | δ ¹H obs. | Multiplicité J (en Hz) | δ ¹³C obs. |
|---|---|---|---|---|---|---|---|
| **1** | 1.10 1.87 | dd(J=4.0-13.0) m | 37.7 | **11** | 1.54 1.66 | m m | 21.2 |
| **2** | 1.52 1.83 | m m | 32.2 | **12** | 1.45 2.00 | m dm(12.0) | 34.4 |
| **3** | 3.54 | tt (4.5-11.0) | 72.0 | **13** | - | - | 44.1 |
| **4** | 2.26 2.32 | ddd(1.5-11.0-13.0) ddd(1.5-4.5-13.0) | 42.7 | **14** | 1.17 | ddd(6.0-10.5-13.0) | 54.6 |
| **5** | - | - | 141.3 | **15** | 1.36 1.81 | m m | 24.0 |
| **6** | 5.37 | d(5.5) | 121.5 | **16** | 2.40 2.50 | dd(8.5-19.0) ddd(1.5-9.0-19.0) | 26.4 |
| **7** | 1.64 2.06 | m ddd(2.5-5.5-12.0) | 31.9 | **17** | - | - | 170.9 |
| **8** | 1.61 | | 31.1 | **18-Me** | 0.93 | s | 17.5 |
| **9** | 1.02 | m | 50.7 | **19-Me** | 1.04 | s | 19.9 |
| **10** | - | - | 37.0 | **OMe** | 3.83 | s | 61.8 |

Le proton en position 3 est axial (3-bêta alcool).

| Numéro | δ ¹H obs. | δ ¹H litt ΔE | δ ^{1H} litt ΔZ | δ ¹³Cobs. | δ ¹³C litt ΔE | δ ¹³C litt ΔZ |
|---|---|---|---|---|---|---|
| **18-Me** | 0.93 | 0.93 | 1.05 | 17.5 | 16.9 | 13.4 |
| **17** | - | - | - | 170.9 | 170.0 | 170.4 |
| **16** | 2.40-2.50 | 2.53-2.52 | 2.42-2.32 | 26.4 | 25.0 | 29.2 |

Obs = observé, litt = d'après la littérature

Spectre de masse : Electro Spray Ionisation en mode positif. MH+= 318.

### 17-β-amino DHEA

On condense 50ml d'ammoniac à -50°C et on ajoute à cette température la solution de 5g de 17-E-méthyloxime de DHEA dans 50ml de tétrahydrofuranne et 7.9 ml d'isopropanol (soit 6.55éq). On ajoute à -50°C, par fractions et sous argon, 1.078g de lithium granules ( soit 9.86 éq). On agite 8 heures à -50°C puis on laisse remonter la température à 20°C. Le produit cristallise en cours de remontée en température. On ajoute 50ml d'eau puis on agite la suspension 2h à 20°C puis on essore et lave à l'eau. On sèche le solide sous vide à 40°C et on obtient 4.55g de 17-β-amino DHEA, soit un rendement de 100%.

Spectre de R.M.N. : 400 MHz, CDCl₃ d1

| Numéro | δ ¹H obs. | Multiplicité J (en Hz) | δ ¹³C obs. | Numéro | δ ¹H obs. | Multiplicité J (en Hz) | δ ¹³C obs. |
|---|---|---|---|---|---|---|---|
| **1** | 1.05 1.87 | m dt(13.5-3.5) | 38.5 | **11** | 1.48 1.61 | m m | 21.8 |
| **2** | 1.48 1.78 | m m | 32.2 | **12** | 1.01 1.83 | m dt(13.0-3.5) | 37.6 |
| **3** | 3.39 | tt (5.5-11.0) | 72.2 | **13** | - | - | 43.3 |
| **4** | 2.17 2.22 | m m | 43.0 | **14** | 0.98 | m | 54.6 |
| **5** | - | - | 142.1 | **15** | 1.21 1.64 | dq(5.5-12.0) m | 24.6 |
| **6** | 5.34 | d (5.0) | 122.1 | **16** | 1.32 1.99 | ddt(2.5-9.0-12.0) m | 31.5 |
| **7** | 1.55 1.99 | m m | 32.7 | **17** | 2.60 | t (9.0) | 63.4 |
| **8** | 1.49 | m | 33.5 | **18-Me** | 0.69 | s | 11.2 |
| **9** | 0.95 | m | 51.8 | **19-Me** | 1.03 | s | 19.9 |
| **10** | - | - | 37.6 | | | | |

Le proton en position 3 est alpha, donc 3-β-alcool.

Le produit 17- bêta amino est pur à 96%. α/β de l'amine primaire = 4/96 Spectre de masse : Electro Spray Ionisation en mode Positif MH⁺ = 290.

### Exemple 12-β-amino Hécogénine

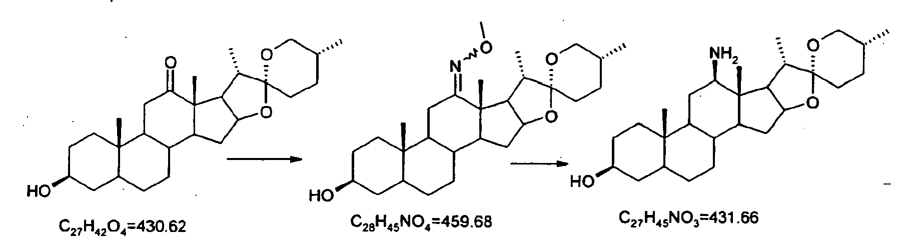

### Préparation de la 12-Z,E-méthyloxime Hécogénine

On dissout à 20°C, 20g d'Hécogénine dans 40ml de toluène et 15.8ml de pyridine (soit 8.9 éq). On ajoute, à 20°C, 2.03g de chlorhydrate de méthoxylamine (soit 1.1 éq). On obtient une suspension blanche que l'on agite 3h à 50°C et 15h à 30°C. On essore et lave à l'eau. On sèche le produit sous vide à 40°C. On obtient 7.05g de 12-Z,E-méthyloxime Hécogénine, soit un rendement de 69.5%.

Spectre de R.M.N. : 400 MHz, CDCl₃ d1

Spectre proton :

| Numéro | δ ¹H obs. | Multiplicité J ( en Hz) | Numéro | δ ¹H obs. | Multiplicité J (en Hz) |
|---|---|---|---|---|---|
| **1** | 1.07 1.76 | M M | **15** | 1.40 2.07 | m m |
| **2** | 1.45 1.85 | M M | **16** | 4.39 | m |
| **3** | 3.60 | tt (5.0 et 11.0) | **17** | 2.56 | dd(6.5-8.5) |
| **4** | 1.32 1.58 | M M | **18-Me** | 0.95 | s |
| **5** | 1.13 | M | **19-Me** | 0.88 | s |
| **6** | 1.61 1.72 | m m | **20** | 1.86 | m |
| **7** | 0.91 1.75 | m m | **21** | 1.10 | d(7.0) |
| **8** | 1.76 | m | **22** | - | - |
| **9** | 0.87 | m | **23** | 1.34 | m |
| **10** | - | - | **24** | 1.47-1.65 | m-m |
| **11** | 1.65 3.16 | m d(5.0- 1 5.0) | **25** | 1.67 | m |
| **12** | - | - | **26** | 3.38 3.50 | t(11.0) ddd(1.5-4.0-11.0) |
| **13** | - | - | **27-Me** | 0.80 | d |
| **14** | 1.35 | m | **OMe** | 3.78 | s |

Spectre carbone :

| Numéro | δ ¹³C obs. | Numéro | δ ¹³C obs. |
|---|---|---|---|
| **1** | 37.0 | **15** | 31.3 |
| **2** | 31.8 | **16** | 80.2 |
| **3** | 71.3 | **17** | 56.3 |
| **4** | 38.5 | **18-Me** | 17.4 |
| **5** | 45.2 | **19-Me** | 12.3 |
| **6** | 32.1 | **20** | 42.7 |
| **7** | 32.4 | **21** | 13.6 |
| **8** | 32.7 | **22** | 109.6 |
| **9** | 54.0 | **23** | 28.8 |
| **10** | 36.4 - | **24** | 29.3 |
| **11** | 21.0 | **25** | 30.7 |
| **12** | 163.8 | **26** | 67.3 |
| **13** | 47.6 | **27-Me** | 17.5 |
| **14** | 56.7 | **OMe** | 61.5 |

19-Me : 12.3 ppm (5α). H en 3 : 3.60ppm ;(il est axial, donc α, car il sort sous forme de triplet avec J = 5.0 et 11.0 Hz.

Spectre de masse : réalisée Electro Spray Ionisation en mode Positif. Présence des 2 isomères Z et E. MH⁺ = 460.

### 12-β-amino Hécogénine

On condense 50ml d'ammoniac à -50°C et on ajoute à cette température la solution de 5g de 17-Z,E-méthyloxime Hécogénine dans 50ml de tétrahydrofuranne et 5.45 ml d'isopropanol (soit 6.5éq). On ajoute sous agitation et sous gaz inerte à -50°C, par fractions et en 3 heures, 0.393g de lithium granules (soit 5.21 éq). On maintient sous agitation pendant ... heures, puis laisse remonter la température à 20°C. On ajoute 50ml d'eau et on distille le tétrahydrofuranne sous vide. On ajoute 100ml d'eau et agite 24h à 20°C. On essore et lave à l'eau. On sèche le solide sous vide à 40°C.

On obtient 4.65g de 12-bêta-amino Hécogénine, soit un rendement de 99%.

Spectre de R.M.N. : 400 MHz, CDCl₃ dl.

Spectre protons :

| Numéro | δ ¹H obs. | Multiplicité J ( en Hz) | δ ¹³C obs. | Numéro | δ ¹H obs. | Multiplicité J (en Hz) | δ ¹³C obs. |
|---|---|---|---|---|---|---|---|
| **1** | 0.98 1.72 | dt(4.0-13.0) m | 37.3 | **15** | 1.37 2.02 | m ddd(6.0-7.0-12.0) | 31.8 |
| **2** | 1.40 1.80 | m m | 32.0 | **16** | 4.43 | ql(7.0) | 81.0 |
| **3** | 3.59 | tt (4.511.0) | 71.5 | **17** | 1.88 | m | 61.8 |
| **4** | 1.29 1.59 | m m | 38.5 | **18-Me** | 0.78 | s | 10.9 |
| **5** | 1.10 | m | 45.2 | **19-Me** | 0.83 | s | 12.9 |
| **6** * | 1.30 1.30 | m m | **29.0** | **20** | 1.88 | m | 42.4 |
| **7** | 0.88 1.69 | m m | **32.5** | **21** | 1.08 | d(7.0) | 14.9 |
| **8** | 1.50 | m | **34.7** | **22** | - | - | 109.6 |
| **9** | 0.78 | m | **54.0** | **23** * | 1.63 1.68 | m m | 32.4 |
| **10** | - | - | **35.9** | **24** | 1.45 1.63 | m-m | 29.3 |
| **11** | 1.22 1.74 | m m | **30.4 25** | | 1.63 | m | 30.6 |
| **12** | 2.58 | d1(10.0) | **61.1** | **26** | 3.36 3.47 | t(11.0) ddd(2.0-4.0-11.0) | 67.2 |
| **13** | - | - | **45.3** | **27-Me** | 0.80 | d (6.0) | 17.6 |
| **14** | 1.09 | m | **56.2** | | | | |

| | | | | | | | |
|---|---|---|---|---|---|---|---|
| * : attributions pouvant être inversées. | | | | | | | |

19-Me : 12.9 ppm, ce qui est caractéristique de la série 5α. H en 3 : 3.59ppm ; il est axial donc α, car il sort sous forme de triplet, avec J = 4.5 et 11.0 Hz.

α/β de l'amine primaire = 5/95

Spectre de masse : Electro Spray Ionisation en mode Positif MH⁺ = 432.

### Exemple 4 : 6-α-amino cholestanol

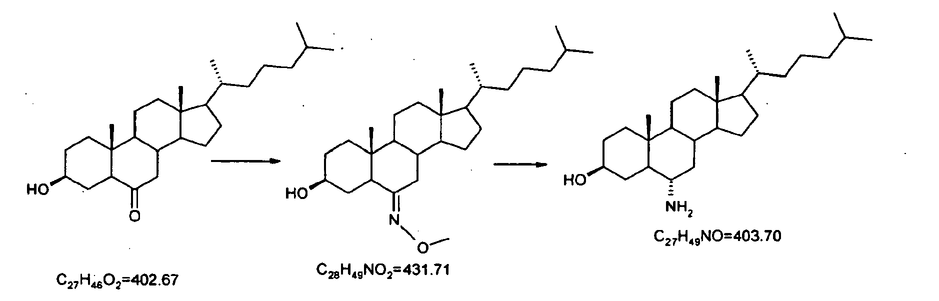

### Préparation du 6-E-méthyloxime cholestanol

On dissout à 20°C, 10g de 6-oxo cholestanol (pureté 99%) dans 40ml de toluène et 17.63 ml de pyridine (soit 8.9 éq). On ajoute, à 20°C, 2.30g de chlorhydrate de méthoxylamine (1.1 éq). On obtient une suspension blanche que l'on agite 6h à 50°C et 30h à 25°C. On ajoute 40ml d'eau, décante et élimine la phase aqueuse. La phase organique est reprise et lavée à l'eau. On distille la solution à sec. Le solide est mis en suspension dans 40ml de n-heptane, essoré et lavé avec 10ml de n-heptane. On sèche le produit sous vide.

On obtient 10.14g de 6-E-méthyloxime cholestanol, soit un rendement de 95.5%.

Spectre de RMN : CDCl₃ : isomère ΔE (OMe cis par rapport au CH₂ en 7) ; H en 3 axial, OH en 3 est donc β (série 5α).

| Numéro | δ ¹H obs. | Multiplicité J( en Hz) | δ ¹³C obs. | Numéro | δ ¹H obs. | Multiplicité J (en Hz) | δ ¹³C obs. |
|---|---|---|---|---|---|---|---|
| **1** | 1.14 1.78 | m dt(13.0-3.5) | 36.5 | **18-Me** | 0.66 | s | 12.5 |
| **2** | 1.41 1.86 | m m | 31.3 | **19-Me** | 0.76 | s | 13.0 |
| **3** | 3.57 | tt(4.5-11.0) | 71.6 | **20** | 1.40 | m | 36.0 |
| **4** | 1.54 2.00 | m m | 32.2 | **21** | 0.91 | d(6.5) | 19.0 |
| **5** | 1.93 | dd(2.5-12.0) | 50.2 | **22** | 1.04 1.36 | m m | 36.7 |
| **6** | - | - | 159.4 | **23** | 1.14 1.34 | m m | 24.2 |
| **7** | 1.20 3.22 | m dd(4.5-13.5) | 30.7 | **24** | 1.14 | m | 40.0 |
| **8** | 1.51 | m | 36.3 | **25** | 1.54 | m | 28.2 |
| **9** | 1.13 | m | 57.0 ou 56.8 | **26 et 27** | 0.86 0.87 | d(6.5) | 22.9 |
| **10** | - | - | 43.2 | **OMe** | 3.82 | s | 61.7 |
| **11** | 1.32 1.57 | m m | 21.8 | | | | |
| **12** | 1.14 2.02 | m m | 40.0 | | | | |
| **13** | - | - | 49.7 | | | | |
| **14** | 0.92 | m | 54.9 | | | | |
| **15** | 1.14 1.62 | m m | 24.4 | | | | |
| **16** | 1.25 1.85 | m m | 28.6 | | | | |
| **17** | 1.13 | m | 56.8 ou 57.0 | | | | |

Spectre de masse : Electro Spray Ionisation en mode Positif. MH⁺ = 432.

### 6-α-amino Cholestanol

On condense 50ml d'ammoniac à -50°C et on ajoute à cette température une suspension de 5g de 6-E-méthyloxime cholestanol dans 50ml de tétrahydrofuranne et 5.80 ml d'isopropanol (soit 6.5éq). On refroidit à -65°C et on ajoute sous agitation, par fractions, en 5 heures, sous argon, 0.490g de lithium granules (soit 6.1 éq). On maintient sous agitation pendant 1 heure, puis laisse remonter la température à 20°C.

On ajoute 50ml d'eau et distille le tétrahydrofuranne sous vide. On essore et lave à l'eau. On sèche le solide sous vide à 40°C et obtient un rendement de 80.2% en 6-alpha-amino cholestanol.

Spectre de RMN : 400MHz, méthanol deutérié.

| Numéro | δ ¹H obs. | Multiplicité J( en Hz) | δ ¹³C obs. | Numéro | δ ¹H obs. | Multiplicité J ( en Hz) | δ ¹³C obs. |
|---|---|---|---|---|---|---|---|
| **1** | 1.00 1.72 | m m | 38.3 | **14** | 1.06 | m | 57.3 |
| **2** | 1.41 1.75 | m m | 31.6 | **15** | 1.19 1.40 | m m | 24.9 |
| **3(axial)** | 3.48 | tt(4.5-11.0) | 71.7 | **16** | 1.30 1.85 | m m | 29.5 |
| **4** | 1.16 1.98 | m dt (12.0-3.5) | 33.2 | **17** | 1.13 | m | 57.5 |
| **5** | 0.91 | m | 52.7 | **18-Me** | 0.69 | s | 12.4 |
| **6(axial)** | 2.55 | dt(3.5-11.0) | 49.9 | **19-Me** | 0.84 | s | 13.7 |
| **7** | 0.77 1.87 | ql(12.0) dt(12.0-3.5) | 42.0 | **20** | 1.39 | m | 36.8 |
| **8** | 1.49 | m | 35.7 | **21** | 0.93 | d(7.0) | 19.0 |
| **9** | 0.70 | m | 55.3 | **22** | 1.03 1.38 | m m | 37.1 |
| **10** | - | - | 36.7 | **23** | 1.13 1.64 | m m | 25.0 |
| **11** | 1.33 1.53 | m m | 22.1 | **24** | 1.16 | m | 40.7 |
| **12** | 1.15 2.02 | m dt(12.0-3.5) | 41.1 | **25** | 1.53 | m | 29.0 |
| **13** | - | - | 43.5 | **26 et 27** | 0.87 0.88 | d(7.0) d(7.0) | 23.0 23.0 |

H en 3 axial, OH en 3 est β (série 5α).
α/β de l'amine primaire = 100/0
Spectre de masse : MH⁺ = 404.

## Revendications

1. Procédé de préparation stéréosélectif d'amines primaires stéroïdes de configuration α ou β, en position 1, 2, 3, 4, 6, 7, 11, 12, 15, 16 ou 17 sur le squelette stéroïde, **caractérisé en ce que** l'on traite un oxime de formule (II) : 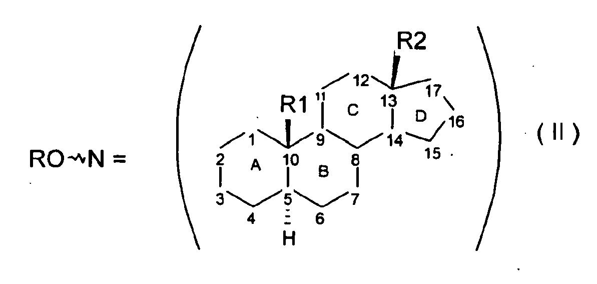 dans laquelle R représente un atome d'hydrogène, un radical alkyle linéaire, ramifié ou cyclique, renfermant de 1 à 12 atomes de carbone ou un radical aryle ou aralkyle renfermant jusqu'à 12 atomes de carbone, R1 représente un atome d'hydrogène ou un radical alkyle inférieur contenant de 1 à 4 atomes de carbone, R2 représente un radical alkyle inférieur contenant de 1 à 4 atomes de carbone, la fonction oxime est située en position 1, 2, 3, 4, 6, 7, 11, 12, 15, 16 ou 17 sur le squelette, lequel peut être substitué par ailleurs par un ou plusieurs groupes non sensibles aux conditions de la réaction définies ci-après, avec du lithium métal dans l'ammoniac liquide, à une 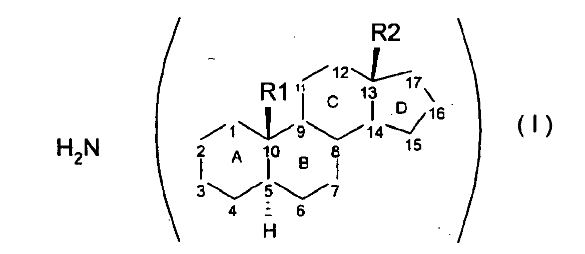 température comprise entre -33°C et -90°C, dans un mélange d'un solvant de type éther et d'un alcool aliphatique, et obtient le composé attendu de formule (I) :
dans laquelle l'amine de configuration α ou β est dans la position correspondant à celle de l'oxime sur le composé de formule (II).

2. Procédé selon la revendication 1, de préparation stéréosélectif d'amines primaires stéroïdes de configuration α ou β, en position 1, 2, 3, 4, 6, 7, 11, 12, 15, 16 ou 17 sur le squelette stéroïde, **caractérisé en ce que** l'on traite un oxime de formule (II) : 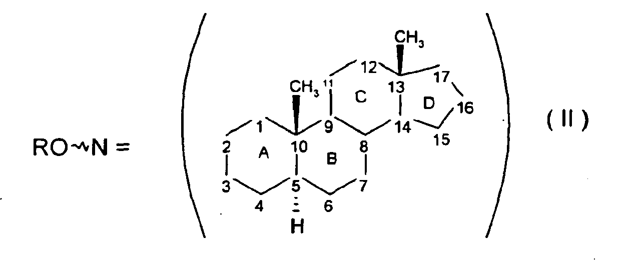 dans laquelle R représente un atome d'hydrogène, un radical alkyle linéaire, ramifié ou cyclique, renfermant de 1 à 12 atomes de carbone ou un radical aryle ou aralkyle renfermant jusqu'à 12 atomes de carbone, la fonction oxime est située en position 1, 2, 3, 4, 6, 7, 11, 12, 15, 16 ou 17 sur le squelette, lequel peut être substitué par ailleurs par un ou plusieurs groupes non sensibles aux conditions de la réaction définies ci-après, avec du lithium métal dans l'ammoniac liquide, à une température comprise entre -33°C et -90°C, dans un mélange d'un solvant de type éther et d'un alcool aliphatique, et obtient le composé attendu de formule (I) : 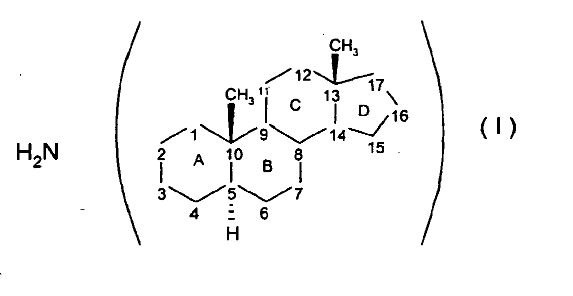 dans laquelle l'amine de configuration α ou β est dans la position correspondant à celle de l'oxime sur le composé de formule (II).

3. Procédé selon la revendication 1 ou 2, **caractérisé en ce que** l'on opère à une température comprise entre -50°C et -80°C.

4. Procédé selon l'une quelconque des revendications 1 à 3, **caractérisé en ce que** l'alcool utilisé est un alcanol renfermant de 1 à 6 atomes de carbone, linéaire, ramifié ou cyclique, éventuellement substitué par un ou plusieurs atomes de fluor.

5. Procédé selon l'une quelconque des revendications 1 à 4, **caractérisé en ce que** l'alcool utilisé est un alcanol renfermant de 1 à 4 atomes de carbone, linéaire ou ramifié, éventuellement substitué par un ou plusieurs atomes de fluor.

6. Procédé selon l'une quelconque des revendications 1 à 5, **caractérisé en ce que** le solvant de type éther utilisé est le tétrahydrofuranne ou le méthyltétrahydrofuranne.

7. Procédé selon l'une quelconque des revendications 1 à 6, **caractérisé en ce que** R représente un radical méthyle, éthyle ou benzyle.

8. Procédé selon l'une quelconque des revendications 1 à 7, **caractérisé en ce que** le squelette du stéroïde de formule (II) mis en jeu est substitué par un ou plusieurs éléments choisis dans le groupe constitué par halogène, cétone libre ou protégée, hydroxy libre ou sous forme éthérifié, amino, carboxy, carboxy estérifié, imide, une chaîne carbonée mono ou divalente saturée ou insaturée, linéaire, ramifiée ou cyclique, renfermant jusqu'à 15 atomes de carbone, le cas échéant interrompue par 1 à 3 atomes d'oxygène, de soufre ou d'azote, et éventuellement substituée par hydroxy ou cétone libre ou protégée, halogène, carboxy, carboxy estérifié et comporte, le cas échéant, une ou plusieurs doubles liaisons dans les cycles A et/ou B et/ou C et/ou D, conjuguées ou non.

9. Procédé selon l'une quelconque des revendications 1 à 8, **caractérisé en ce que** lorsque le stéroïde est substitué par une chaîne alcoylène, celle-ci n'est pas un méthylène en 17.

10. Procédé selon l'une quelconque des revendications 1 à 9, **caractérisé en ce que** le squelette du stéroïde mis en jeu est substitué par un ou plusieurs éléments choisis dans le groupe constitué par fluor, cétone libre ou protégée, hydroxy libre ou protégé, amino, éther, amide, imide, les chaînes alcoyle, alcényle, alcynyle et alcoylène telles que définies plus haut, et comporte, le cas échéant, une ou plusieurs doubles liaisons dans les cycles A et/ou B et/ou C et/ou D, conjuguées ou non.

11. Procédé selon l'une quelconque des revendications 1 à 9, **caractérisé en ce que** le squelette du stéroïde mis en jeu est substitué par un ou plusieurs éléments choisis dans le groupe constitué par cétone libre ou protégée, hydroxy libre ou protégé et une chaîne alcoyle linéaire ou ramifiée, renfermant jusqu'à 12 atomes de carbone, et comporte, le cas échéant, une ou deux doubles liaisons dans les cycles A et/ou B et/ou C et/ou D.

## Claims

1. Process for the stereoselective preparation of steroid primary amines of α- or β-configuration, at position 1, 2, 3, 4, 6, 7, 11, 12, 15, 16 or 17 on the steroid backbone, **characterized in that** an oxime of formula (II): 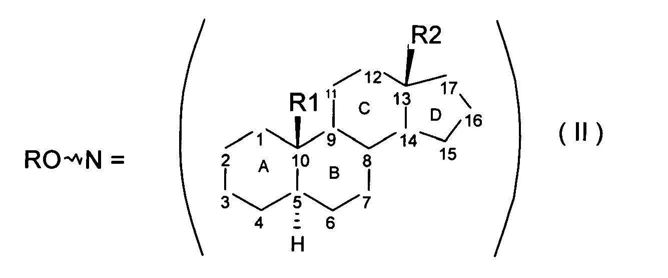 in which R represents a hydrogen atom, a linear, branched or cyclic alkyl radical containing from 1 to 12 carbon atoms, or an aryl or aralkyl radical containing up to 12 carbon atoms, R1 represents a hydrogen atom or a lower alkyl radical containing from 1 to 4 carbon atoms, R2 represents a lower alkyl radical containing from 1 to 4 carbon atoms, the oxime function is located at position 1, 2, 3, 4, 6, 7, 11, 12, 15, 16 or 17 on the backbone, which can otherwise be substituted with one or more groups not sensitive to the reaction conditions defined hereinafter, is treated with lithium metal in liquid ammonia, at a temperature of between -33°C and -90°C, in a mixture of a solvent
of ether type and of an aliphatic alcohol, and the expected compound of formula (I): 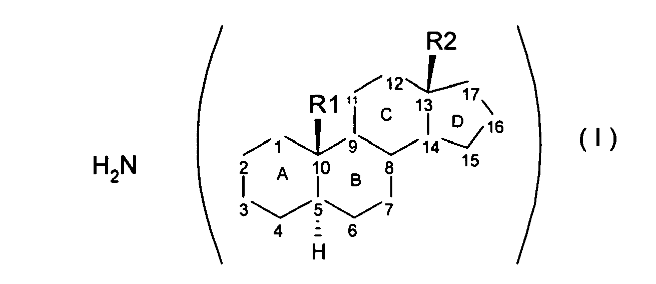 in which the amine of α- or β-configuration is in the position corresponding to that of the oxime on the compound of formula (II), is obtained.

2. Process according to Claim 1, for the stereoselective preparation of steroid primary amines of α- or β-configuration, at position 1, 2, 3, 4, 6, 7, 11, 12, 15, 16 or 17 on the steroid backbone, **characterized in that** an oxime of formula (II): 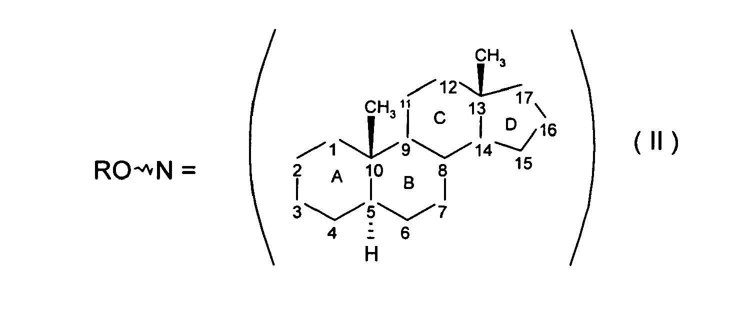 in which R represents a hydrogen atom, a linear, branched or cyclic alkyl radical containing from 1 to 12 carbon atoms, or an aryl or aralkyl radical containing up to 12 carbon atoms, the oxime function is located at position 1, 2, 3, 4, 6, 7, 11, 12, 15, 16 or 17 on the backbone, which can be otherwise substituted with one or more groups not sensitive to the reaction conditions defined hereinafter, is treated with lithium metal in liquid ammonia, at a temperature of between -33°C and -90°C, in a mixture of a solvent of ether type and of an aliphatic alcohol, and the expected compound of formula (I): 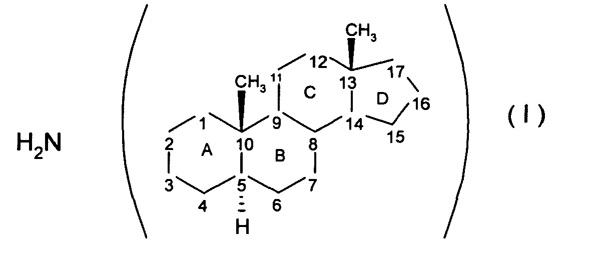 in which the amine of α- or β-configuration is in the position corresponding to that of the oxime on the compound of formula (II), is obtained.

3. Process according to Claim 1 or 2, **characterized in that** it is carried out at a temperature of between -50°C and -80°C.

4. Process according to any one of Claims 1 to 3, **characterized in that** the alcohol used is a linear, branched or cyclic alkanol containing from 1 to 6 carbon atoms, optionally substituted with one or more fluorine atoms.

5. Process according to any one of Claims 1 to 4, **characterized in that** the alcohol used is a linear or branched alkanol containing from 1 to 4 carbon atoms, optionally substituted with one or more fluorine atoms.

6. Process according to any one of Claims 1 to 5, **characterized in that** the solvent of ether type used is tetrahydrofuran or methyltetrahydrofuran.

7. Process according to any one of Claims 1 to 6, **characterized in that** R represents a methyl, ethyl or benzyl radical.

8. Process according to any one of Claims 1 to 7, **characterized in that** the backbone of the steroid of formula (II) involved is substituted with one or more elements chosen from the group consisting of halogen, free or protected ketone, hydroxyl in free or etherified form, amino, carboxyl, esterified carboxyl, imide, and a saturated or unsaturated, linear, branched or cyclic, monovalent or divalent carbon chain containing up to 15 carbon atoms, where appropriate interrupted with 1 to 3 oxygen, sulphur or nitrogen atoms, and optionally substituted with hydroxyl or ketone which may be free or protected, halogen, carboxyl or esterified carboxyl, and comprises, where appropriate, one or more double bonds in the A and/or B and/or C and/or D rings, which may or may not be conjugated.

9. Process according to any one of Claims 1 to 8, **characterized in that**, when the steroid is substituted with an alkylene chain, the latter is not a methylene in the 17-position.

10. Process according to any one of Claims 1 to 9, **characterized in that** the steroid backbone involved is substituted with one or more elements chosen from the group consisting of fluorine, free or protected ketone, free or protected hydroxyl, amino, ether, amide, imide, and alkyl, alkenyl, alkynyl and alkylene chains as defined above, and comprises, where appropriate, one or more double bonds in the A and/or B and/or C and/or D rings, which may or may not be conjugated.

11. Process according to any one of Claims 1 to 9, **characterized in that** the steroid backbone involved is substituted with one or more elements chosen from the group consisting of free or protected ketone, free or protected hydroxyl, and a linear or branched alkyl chain containing up to 12 carbon atoms, and comprises, where appropriate, one or two double bonds in the A and/or B and/or C and/or D rings.

## Patentansprüche

1. Verfahren zur stereoselektiven Herstellung primärer Steroidamine mit α- oder β-Konfiguration an Position 1, 2, 3, 4, 6, 7, 11, 12, 15, 16 oder 17 am Steroidgrundgerüst, **dadurch gekennzeichnet, dass** man ein Oxim der Formel (II): 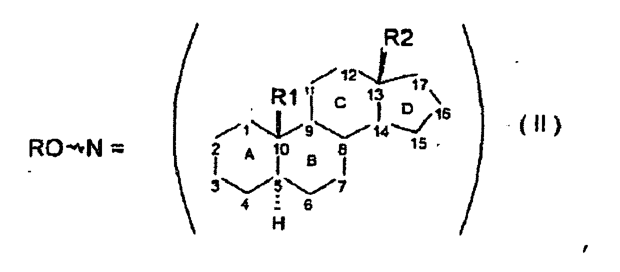 worin R für ein Wasserstoffatom, einen geraden, verzweigten oder cyclischen Alkylrest mit 1 bis 12 Kohlenstoffatomen oder einen Aryl- oder Aralkylrest mit bis zu 12 Kohlenstoffatomen steht, R1 für ein Wasserstoffatom oder einen Niederalkylrest mit 1 bis 4 Kohlenstoffatomen steht, R2 für einen Niederalkylrest mit 1 bis 4 Kohlenstoffatomen steht, wobei sich die Oximfunktion an Position 1, 2, 3, 4, 6, 7, 11, 12, 15, 16 oder 17 des Grundgerüsts befindet, das außerdem mit einer oder mehreren Gruppen substituiert sein kann, die gegenüber den nachstehend definierten Bedingungen der Reaktion nicht empfindlich sind, mit Lithiummetall in flüssigem Ammoniak bei einer Temperatur zwischen -33°C und -90°C in einem Gemisch aus einem Lösungsmittel des Ethertyps und einem aliphatischen Alkohol behandelt und die erwartete Verbindung der Formel (I) erhält: 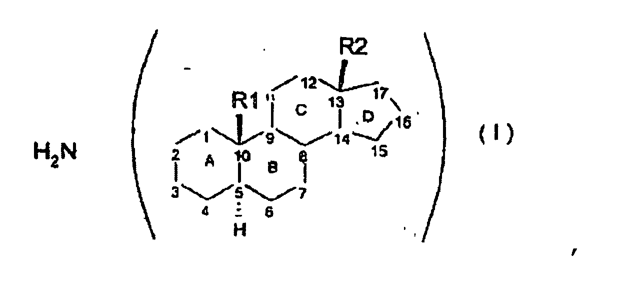 in der das Amin mit der α- oder β-Konfiguration sich an der Position befindet, die derjenigen des Oxims in der Verbindung der Formel (II) entspricht.

2. Verfahren nach Anspruch 1 zur stereoselektiven Herstellung primärer Steroidamine mit α- oder β-Konfiguration an Position 1, 2, 3, 4, 6, 7, 11, 12, 15, 16 oder 17 am Steroidgrundgerüst, **dadurch gekennzeichnet, dass** man ein Oxim der Formel (II): 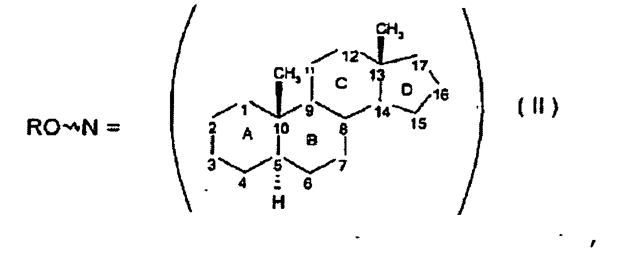 worin R für ein Wasserstoffatom, einen geraden, verzweigten oder cyclischen Alkylrest mit 1 bis 12 Kohlenstoffatomen oder einen Aryl- oder Aralkylrest mit bis zu 12 Kohlenstoffatomen steht, wobei sich die Oximfunktion an Position 1, 2, 3, 4, 6, 7, 11, 12, 15, 16 oder 17 des Grundgerüsts befindet, das außerdem mit einer oder mehreren Gruppen substituiert sein kann, die gegenüber den nachstehend definierten Bedingungen der Reaktion nicht empfindlich sind, mit Lithiummetall in flüssigem Ammoniak bei einer Temperatur zwischen -33°C und -90°C in einem Gemisch aus einem Lösungsmittel des Ethertyps und einem aliphatischen Alkohol behandelt und die erwartete Verbindung der Formel (I) erhält: 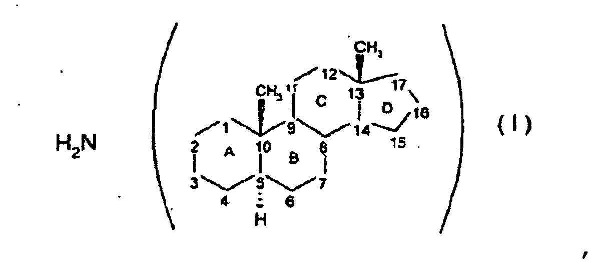 in der das Amin mit der α- oder β-Konfiguration sich an der Position befindet, die derjenigen des Oxims in der Verbindung der Formel (II) entspricht.

3. Verfahren nach Anspruch 1 oder 2, **dadurch gekennzeichnet, dass** man bei einer Temperatur zwischen -50°C und -80°C arbeitet.

4. Verfahren nach einem der Ansprüche 1 bis 3, **dadurch gekennzeichnet, dass** der verwendete Alkohol ein gerades, verzweigtes oder cyclisches Alkanol mit 1 bis 6 Kohlenstoffatomen ist, das gegebenenfalls mit einem oder mehreren Fluoratomen substituiert ist.

5. Verfahren nach einem der Ansprüche 1 bis 4, **dadurch gekennzeichnet, dass** der verwendete Alkohol ein gerades oder verzweigtes Alkanol mit 1 bis 4 Kohlenstoffatomen ist, das gegebenenfalls mit einem oder mehreren Fluoratomen substituiert ist.

6. Verfahren nach einem der Ansprüche 1 bis 5, **dadurch gekennzeichnet, dass** das verwendete Lösungsmittel des Ethertyps Tetrahydrofuran oder Methyltetrahydrofuran ist.

7. Verfahren nach einem der Ansprüche 1 bis 6, **dadurch gekennzeichnet, dass** R für einen Methyl-, Ethyl- oder Benzylrest steht.

8. Verfahren nach einem der Ansprüche 1 bis 7, **dadurch gekennzeichnet, dass** das verwendete Steroidgrundgerüst der Formel (II) mit einem oder mehreren Elementen substituiert ist, die ausgewählt sind aus der Gruppe, bestehend aus Halogen, freiem oder geschütztem Keton, Hydroxy in freier oder veretherter Form, Amino, Carboxy, verestertem Carboxy, Imid, einer ein- oder zweiwertigen, gesättigten oder ungesättigten, geraden, verzweigten oder cyclischen Kohlenstoffkette mit bis zu 15 Kohlenstoffatomen, die gegebenenfalls durch 1 bis 3 Sauerstoff-, Schwefel- oder Stickstoffatome unterbrochen ist und gegebenenfalls mit freiem oder geschütztem Hydroxy oder Keton, Halogen, Carboxy, verestertem Carboxy substituiert ist, und gegebenenfalls eine oder mehrere konjugierte oder nicht konjugierte Doppelbindungen in den Ringen A und/oder B und/oder C und/oder D aufweist.

9. Verfahren nach einem der Ansprüche 1 bis 8, **dadurch gekennzeichnet, dass**, wenn das Steroid mit einer Alkylenkette substituiert ist, diese kein Methylen an Position 17 ist.

10. Verfahren nach einem der Ansprüche 1 bis 9, **dadurch gekennzeichnet, dass** das verwendete Steroidgrundgerüst mit einem oder mehreren Elementen substituiert ist, die ausgewählt sind aus der Gruppe, bestehend aus Fluor, freiem oder geschütztem Keton, freiem oder geschütztem Hydroxy, Amino, Ether, Amid, Imid, Alkyl-, Alkenyl-, Alkinyl- und Alkylenketten, wie vorstehend definiert, und gegebenenfalls eine oder mehrere konjugierte oder nicht konjugierte Doppelbindungen in den Ringen A und/oder B und/oder C und/oder D aufweist.

11. Verfahren nach einem der Ansprüche 1 bis 9, **dadurch gekennzeichnet, dass** das verwendete Steroidgrundgerüst mit einem oder mehreren Elementen substituiert ist, die ausgewählt sind aus der Gruppe, bestehend aus freiem oder geschütztem Keton, freiem oder geschütztem Hydroxy und einer geraden oder verzweigten Alkylkette mit bis zu 12 Kohlenstoffatomen, und gegebenenfalls eine oder zwei Doppelbindungen in den Ringen A und/oder B und/oder C und/oder D aufweist.
